# EUROPEAN PATENT SPECIFICATION

(11) **EP 4 054 509 B1**
(45) Date of publication and mention of the grant of the patent: **17.01.2024**
(21) Application number: 20823982.2
(22) Date of filing: 09.11.2020
(51) Int. Cl.: A61J 1/20, A61J 3/00, A61M 5/14

(54) **ALTERNATIVE FLUIDIC PATHING FOR SERIALLY CONNECTABLE DRUG MODULES**
ALTERNATIVE FLUIDISCHE VORRICHTUNG FÜR SERIELL VERBINDBARE ARZNEIMITTELMODULE
CHEMIN FLUIDIQUE ALTERNATIF POUR MODULES DE MÉDICAMENT POUVANT ÊTRE CONNECTÉS EN SÉRIE

(30) Priority: 08.11.2019 US 201962932993 P
(43) Date of publication of application: 14.09.2022
(60) Divisional application: 23214945.0
(73) Proprietor: Bristol-Myers Squibb Company, Princeton, NJ 08543 (US)
(72) Inventor: MCLOUGHLIN, Martin John, Princeton, NJ 08543 (US); ZIEMINSKI, Stephen Lawrence, Princeton, NJ 08543 (US); HOWANSKY, Mark Steven, Princeton, NJ 08543 (US); BERARDOCCO, Frank, Princeton, NJ 08543 (US)
(74) Representative: White, Andrew John
(86) International application number: PCT/US2020/059721
(87) International publication number: WO 2021/092578

(56) References cited:
- WO-A1-2016/205687
- WO-A1-2019/217864
- WO-A2-2007/008593
- WO-A2-2007/107406
- US-A1- 2008 164 273

## Description

### Field of the Invention

The field of the present invention is the compounding and preparation of liquid drugs, especially for intra-venous infusion and direct patient infusion. More particularly the invention relates to solutions for fluidic circuitry and connections that would enable the application of devices for the preparation and compounding of combinations of two or more drugs.

### Background to the Invention

It is common practice in the administration of drugs by intravenous infusion for the drugs to be compounded within a pharmacy environment. Such drugs are typically supplied sterile in glass vials and may be supplied in solid or aqueous solution form. When supplied in solid form the drugs must be reconstituted with a sterile aqueous diluent prior to transfer to the infusion bag. The person skilled in the art will appreciate that such drug formulations will typically include several excipients for example buffers, pH modifiers, tonicity modifiers, stabilizers and so on. Typically liquid drugs for intra-venous infusion are compounded in an infusion bag in a pharmacy environment prior to transfer to the patient for infusion. Because of the need to maintain sterility of the drugs while compounding the compounding procedure is typically performed in an aseptic pharmacy hood. Typically, the pharmacist or pharmacy technician (practitioner) will prepare the drugs in accordance with an individual patient prescription.

After ensuring the hood is clear of all materials the practitioner will retrieve, vials of the drugs required per the prescription from the pharmacy stocks and will verify their identity and strength. The verification process may be assisted by use of a bar code scanner or other identification technology. The practitioner will also pick from stock all of the other necessary equipment required to safely prepare the drugs for infusion including the infusion bag itself, syringes, needles, transfer sets, gloves, sharps disposal containers and so on. Once all of the necessary equipment has been assembled the practitioner will follow a protocol for the preparation of the drugs which may include the reconstitution of solid drugs by addition of diluents, the ordered withdrawal of liquid drugs from their individual vials into the IV bag via the transfer port. Typically this procedure is performed manually and involves the use of multiple needles. The risk of needle-stick injuries to the practitioner is increased by each needle required to effect the compounding of the drugs. With high potency or toxicity drugs, e.g. cytotoxic agents for chemotherapy, this presents a considerable exposure risk for the practitioner.

To eliminate some of the risks associated with manual preparation including exposure to dangerous drugs and the risk of medication errors, pharmacy compounding machines are known to the person skilled in the art which automate many of the steps involved in the preparation and compounding of drugs. Typically such machines are complex electromechanical systems which implement sophisticated precision dispensing mechanisms for the accurate reconstitution of liquid drugs. Aside from their cost, size and complexity, many of the designs for such machines described in the art draw liquid drugs from a stock reservoir and so only use a fraction of the drug in the container. Because of the need to maintain sterility, unused drug solutions must typically be discarded and so are wasted. With the very high cost of some drugs, especially biologic drugs, this waste is a significant undesirable cost. When the wasted drugs are cytotoxic agents, their disposal creates a significant environmental and safety hazard.

Recent advances in medicine, particularly in the treatment of cancer, have demonstrated that therapeutically beneficial effects can be achieved by the synergistic combination of two or more drugs.

For example, recent clinical research has demonstrated that the combination of an anti-PD-1 checkpoint inhibitor drug with a CTLA4 checkpoint inhibitor can have beneficial synergistic effects in some tumor types which can lead to better clinical outcomes than could be achieved by the individual administration of either drug alone. Typically such checkpoint inhibitor drugs are biotechnology derived monoclonal antibodies or fragments thereof of the immunoglobulin type. In some situations it may be beneficial to combine such biologic drugs with conventional chemotherapy agents such as cytotoxic drugs.

Applicant has now realized that the combinatorial principles described in U.S. Provisional Patent Application No. 62/670,266, filed on May 11, 2018, PCT Appl. No. PCT/US2019/031727, filed May 10, 2019, PCT Appl. No. PCT/US2019/031762, filed May 10, 2019, and PCT Appl. No. PCT/US2019/031791, filed May 10, 2019, to the same assignee as herein, can address several of the challenges encountered in the preparation and compounding of drugs for intra-venous infusion and can provide several advantages including but not limited to simplification of pharmacy procedures, reduction in the risk of medication errors, containment and protection for the practitioner from highly potent or highly toxic agents, reduction in the risk of needle-stick injuries, reduction or elimination of drug waste, avoidance of the need for complex and expensive pharmacy compounding machines. As a consequence of these advantages in embodiments the present invention may further enable the preparation and compounding of drugs for IV infusion at locations remote from the pharmacy, and by a non-specialist practitioner, for example by a suitably trained technician or nurse at the patient's home. This possibility is enhanced by the intrinsic portability of the system described herein.

WO2016/205687 describes a device for pooling a fluid from a container unit having at least one container, and includes an inlet port having at least one inlet channel configured for receiving the fluid or ambient air, and an outlet port having at least one outlet channel configured for delivering the fluid to an attachment. WO2007/107406 describes a device for delivering a liquid drug to a user. The device comprises a reservoir adapted to contain a liquid, an outlet opening for delivering liquid drug from the device, and means for connecting the device to at least one other at least substantially identical device. WO2019/217864 describes a combinatorial drug delivery device for delivering a predetermined selection of drug components. The device includes a plurality of modules, each including: a body having an interior volume; a spike plate movably disposed in the interior volume, the spike plate having a protruding cannula and first and second ports. WO2007/008593 describes a system that allows for the safe, rapid, efficient recovery of a drug solution from sealed vials. The system is closed so that highly potent compounds can later be recovered and reworked without large investment in further engineering controls. US2008/164273 describes a drug storage and dispensing system including a container for containing the drug, a storage tray and a drug identification key.

### Summary of the Invention

Aspects of the invention are as set out in the appended claims. According to the present disclosure, there are provided drug modules each defining a chamber for the receipt of a drug filled vial. In embodiments, spacing adapters may be provided, or vial-retaining dimensions may be altered, to enable the receipt of different sized vials. The modules may further comprise a displaceable vial retainer to allow access to the vial septum for disinfection, a cannula arranged to breach the vial septum on displacement of the vial retainer, sterile tubing defining a sterile fluid path from the cannula to the inlet and outlet ports which connect the sterile fluid paths on adjacent modules. The modules also may comprise male and female mating features such that any number of modules of identical design can attach to each other in a 'stack'. Further the mating features and ports may be arranged so that the fluidic connections between the modules are made automatically when modules are serially connected together via their respective mating features. Each module may be also provided with a vent, including a vent terminated by an aseptic particulate filter that enables the equalization of pressure within the vial during removal of liquid drug from the vial whilst preventing the entrainment of contaminants into the fluid path. The vents may be arranged such that when another module is mated adjacent to the vent side of the module, a seal is formed which blocks that vent. In this way, only the terminal module on a stack may be vented to atmosphere.

In embodiments, the system could be equipped with a sterile air reservoir, which would be used in place of atmospheric air. This feature would enable the system to be used in less controlled environments, specifically within the patient home setting.

The first module in the stack may be connected to a housing which comprises a male port similar to that provided on the modules. The housing comprises further sterile tubing, which may extend from the housing and be terminated by a sterile hollow needle which may be used to breach the sterile port on an infusion bag or other container for the pumped transfer of liquid drugs into the bag or other container, or may be used for drug administration directly into a patient. Further pumping means may be provided so that when fully connected the liquid drugs in the vials may be pumped as one from their respective vials to the needle.

In embodiments such pumping means may be integral to the housing or may be external to the housing. The pumping means may be sterile and form a component of the fluid path or may be of the non-contacting variety such as peristaltic pumps. The person skilled in the art will be familiar with several pumping technologies suitable for use in the pumped transfer of liquid drugs in the manner described.

In embodiments of this delivery system, allowing modules the ability to not only connect with each other but also be configured with a common base tray brings additional flexibility to the system. The base tray allows a low-cost alternative to control the order and assembly of the modules without relying on more complex an electronic error prevention systems, which would be necessary if the modules are to be configured in a purely stackable architecture.

In current embodiments of this system, which use a base tray, drug modules, are inserted and mechanically locked into a provided common base tray, which fluidically connects all inserted modules and delivers all modules' contents. The base tray consists of several prearranged wells designed to accept the hinged cap geometry of the modules. Within each well are two check valve styled fluidic connection ports that interface with the input and output ports of the module vial spike plate. By sufficiently pressing the vial spike plate into the base tray's valves, a fluidic connection is made between the base tray valves through the vial spike plate. Internally, sterile tubing is used to fluidically connect a single check valve in adjacent wells to create a fluidic circuit, which is only completed once all the wells of the base tray have been filled with the specified modules. The base tray will contain an air filter and/or vent which will terminate one end of the internal fluidic circuit. The fluidic circuit of the base tray will flow the drug product from the modules out a sterilized main output line from the base tray. A one-way valve in the main output line will prevent drug product from back flushing into the base tray.

Cooperating mechanical keying features present on both the internal walls of the base tray wells and the outer surface of the module's hinged cap create a poka yoke mechanism that will ensure that modules may only be inserted into prescribed wells of the base tray.

The main output line from the base tray may be terminated by a sterile hollow needle which may be used to breach the sterile port on an infusion bag or other container for the pumped transfer of liquid drugs into the container or may be used for drug administration directly into a patient. Further, a pump may be provided so that, when fully connected, the liquid drugs in the vials may be pumped as one collective output from their respective vials into the bag, container, or patient.

The pump may be integral to the base tray or may be external to the base tray. The pump maybe sterile and form a component of the fluid path or may be of the non-contacting variety such as peristaltic pumps. The person skilled in the art will be familiar with several pumping technologies suitable for use in the pumped transfer of liquid drugs in the manner described.

Alterations to either the module or the tray fluidic architectures could be made to the current described systems, which would enable the modules to be configured in a stackable arrangement or nested within a base tray. The configuration of these systems can then be made on a case-by-case basis either at the point of component assembly, in supply chain, or by the user at the point-of-care.

### Figure Descriptions (Figures 5a, 5b, 6a, 6b, 8, 9, 10 and 11 do not correspond to embodiments of the invention)

Figure 1- Dual Circuit Module
Figure 2- Dual Circuit Modules connected
Figure 3- Dual Circuit Modules connected with pump
Figure 4- Dual Circuit Modules with sterile air reservoir
Figures 5a and 5b - Configurable Module Housing
Figures 6a and 6b- Translatable Module Ports
Figure 7- Redundant Module Fluidics
Figure 8- Side loading tray
Figure 9- Vial float valve
Figure 10- Collapsible Tray
Figure 11- Module Tray adapter

### Detailed Description

Various embodiments are described herein related to modules useable with combinatorial drug delivery devices, including devices formed by serially connecting a plurality of the modules (e.g., to form a stack), and devices formed by mounting the modules to a base tray, or other support structures, which fluidically connects the modules. Features of the embodiments, even if not expressly disclosed, are combinable in various combinations. Explanations of certain features, e.g., the spike plate, the interaction of the spike plate and a mounted vial, venting, and so forth, are provided with certain embodiments, are equally applicable to other embodiments, as will be appreciated by those skilled in the art.

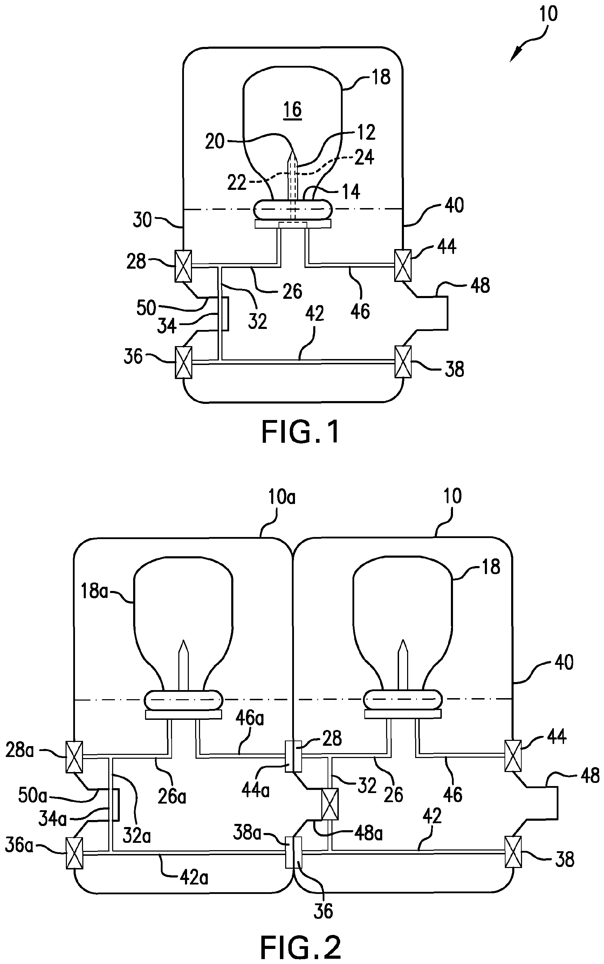

In a first embodiment, as depicted in Figures 1-4, a module 10 may be provided with a vial spike 12 used to pierce a vial septum 14 extending into the interior volume 16 of a liquid filled drug vial 18. A distal end 20 of the vial spike 12 may be sharpened to facilitate piercing of the vial septum 14. The vial spike 12 must be provided with sufficient length to fully pierce the septum 14 in accessing the interior volume 16. The vial spike 12 includes two lumens dividing the module fluidics into separate circuits, an inlet path 22, and an outlet path 24. With the vial spike 12 piercing the septum, the inlet path 22 and the outlet path 24 are in communication with the interior volume 16 of the drug vial 18 via openings formed in the distal end 20 of the vial spike 12.

A first fluid line 26 extends from the inlet path 22 to a first sealing port 28, located on a back face 30 of the module 10, to connect the interior volume 16 of the drug vial 18 with the first sealing port 28. A first branch line 32 extends from, and is in communication with, the first fluid line 26. The first branch line 32 includes an exposed tube portion 34, which is exposed through the back face 30 of the module 10. It is preferred that the exposed tube portion 34 be formed by flexible tubing. A second sealing port 36 is located on the back face 30 of the module 10 connected to a third sealing port 38, located on a front face 40 of the module 10, by a second fluid line 42. The first branch line 32 extends to, and is in communication with, the second fluid line 42. A fourth sealing port 44 is located on the front face 40 which is connected to the outlet path 24 by a third fluid line 46. A male pin 48 protrudes from the front face 40 configured to pressingly engage against the exposed tube portion 34 of a similarly-formed module connected to the module 10, as discussed below. A port or opening 50 may be formed in the back face 30 formed to expose the exposed tube portion 34 and to receive the male pin 48.

Figures 2-4 show two of the modules 10 being serially connected. As will be appreciated by those skilled in the art, any quantity of the modules 10 may be utilized in serial connection. This allows for the drug vials 18 of the modules 10 to contain different drugs (type, concentration) which may be drawn in sequence and administered as a combination. For illustrative purposes, a second of the modules 10 is designated with the same reference numbering as used with the drug module 10 but with the additional designation with the letter "a". As the modules 10, 10a are connected, as shown in Figure 2, the first and second sealing ports 28, 36 on the back face 30 of the module 10 interface with the third and fourth sealing ports 38a, 44a of the second module 10a making fluid connections between primary and secondary circuits of the modules 10, 10a. The male pin 48a on the second module 10a is received in the port 50 to interface with the exposed tube portion 34, particularly to pressingly engage the exposed tube portion 34 in pinching closed the flexible tubing thereof, thereby connecting the primary and secondary input circuits of the series of the modules 10, 10a through the last module 10a. In particular, a fluidic chain is made between the modules 10, 10a where the primary and secondary input circuits are in communication by being bridged by the first branch line 32a with the exposed tube portion 34a being open to flow therethrough. This arrangement allows for the first branch lines 32 of the modules 10 to be sealed, except for the ultimate module 10a, where flow is permitted to close the fluidic circuit. Thus, the fluidic pathway may begin at the third sealing port 38 of the first module 10, continue through the second fluid line 42 of each of the modules 10, 10a, through the first branch line 32a of the last module 10a of the chain, then return back through the drug vial 18, 18a of each of the modules 10, 10a, and at the fourth sealing port 44 on the front face 40 of the first module 10.

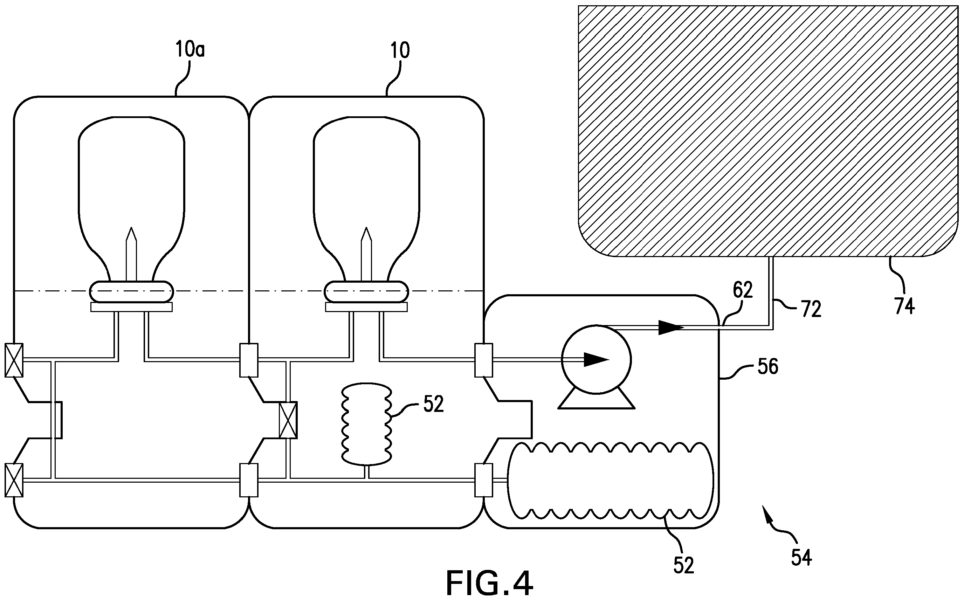

As will be understood by those skilled in the art, the fluidic pathway through the modules 10, 10a may be vented as in any known manner. For example, one or both of the first and second sealing ports 28, 36 may be provided with an anti-microbial filter which allows flow of air therethrough but restricts passage of microbes therethrough. In addition, or alternatively, as shown in Figure 4, one or more collapsible gas chambers 52, e.g., to accommodate a sterile gas (e.g., sterile air) or a stable gas (e.g., argon), may be provided in communication with any of the fluid lines, including the first fluid line 26, the second fluid line 42, and the third fluid line 46. In addition, the fluid lines (the first fluid line 26, the first branch line 32, the second fluid line 42, the third fluid line 46) may be wholly or partially defined by tubing, including flexible tubing. In addition, or alternatively, the fluid lines (the first fluid line 26, the first branch line 32, the second fluid line 42, the third fluid line 46) may be wholly or partially formed by passages defined in the modules 10. As indicated above, it is preferred that the exposed tube portion 34 by defined by a flexible tube to be pinchable in response to pressing engagement by the male pin 48 of an adjacent, connected module 10.

In addition, the modules 10 may be connected in any known manner, e.g., including cooperating locking elements on opposing faces. Interengagement of the male pin 48 with the port 50 between adjacent modules 10 may be utilized to form a connection therebetween.

With all the modules 10 serially connected in fluidic communication, to form a drug delivery device 54, a pump module 56 may be placed on the front face 40 of the first module 10 in the chain to interface with the third and fourth sealing ports 38, 44 as input and output ports of the module chain. The pump module 56 includes a first inlet sealing port 58, formed to interengage with the fourth sealing port 44 of the first module 10, a second inlet sealing port 60, formed to interengage with the third sealing port 38 of the first module 10, a first outlet port 62, and, a pump 64. An outlet passageway 66 is defined between the first inlet sealing port 58 and the first outlet port 62, with the pump 64 being configured to draw liquid drug into the outlet passageway 66, via the fourth sealing port 44/the first inlet sealing port 58 interface, and to urge the drawn liquid drug from the first outlet port 62. The outlet passageway 66 may be a closed passageway with the pump 64 being peristaltic, acting on the outlet passageway 66, to maintain sterility of the liquid drug passing therethrough.

A vented passageway 68 may be provided in communication with the second inlet sealing port 60 to provide venting to the fluidic circuit of the modules 10, 10a, via the third sealing port 38. The vented passageway 68 may terminate at a one-way air vent 70. As shown in Figure 4, the one-way air vent 70 may be substituted with one or more collapsible gas chambers 52, e.g. containing sterile gas (e.g., sterile air) or stable gas (e.g., argon). This would allow the device 54 to be utilized in a home care setting where access to controlled environment with air-filtration may not be available.

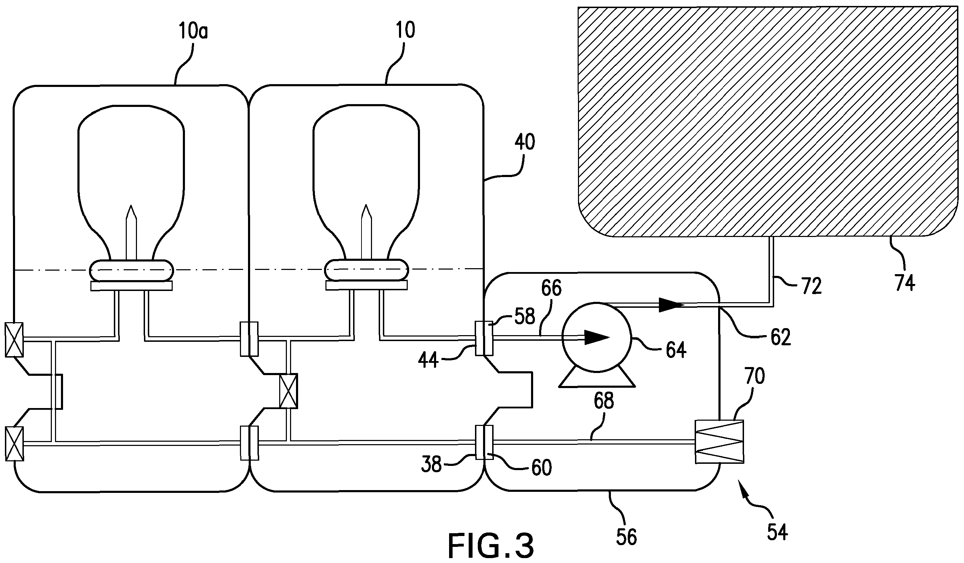

The drug delivery device 54 may be utilized in various applications. The drug delivery device 54 is particularly well-suited for in-home use, being self-contained to provide combination drug therapy. The first outlet port 62 may be connected to flexible tubing 72, connected in turn with a drug delivery needle (not shown) for direct drug administration to a patient or connected in turn with a container, such as an IV bag 74, in which the drug may collect for subsequent administration. In use, as shown in Figures 3 and 4, the pump 64 may be actuated to draw the liquid drug from the drug vials 18, 18a to be delivered via the first outlet port 62, with possible venting provided by one or more of the collapsible gas chambers 52 and/or the vented passageway 68.

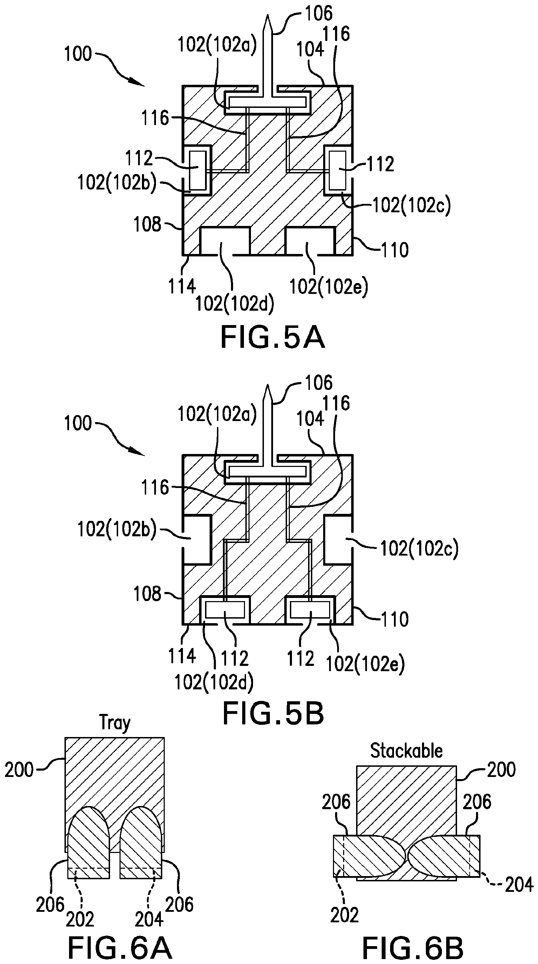

Alternate embodiments of the module fluidics may be desirable in allowing the capability to use configurable modules to allow for being serially connected together or, alternatively, configured for mounting to a base tray, or other supporting structure, such as a "poke yoke" tray. As depicted in Figures 5a and 5b, cross-sections of a module body housing 100 are shown with cutouts 102 for securing different components of a fluidic path subassembly. A top face 104 of the housing 100, has a cutout 102a for a spike plate 106, formed for accessing drug from a drug vial in similar manner to that described above, while on front and back faces 108, 110, each have a cutout 102b, 102c for the securement of a sealing port 112, a bottom face 114 having cutouts 102d, 102e for two sealing ports 112. The spike plate 106 is a two-lumen spike plate where each lumen continues to a piece of flexible tubing 116 which terminates at a sealing port 112. At the point of module assembly, the spike plate 106 may be assembled into the top cutout 102a with sealing ports 112 being arranged as the inlet and outlet of the fluidic path by being placed in either the opposing front and back faces 108, 110 of the module housing 100 (Figure 5a), for a serially stackable configuration, or, on the bottom face 114 (Figure 5b), permitting interface of the module 100 with a base tray or other supporting structure.

An additional embodiment of this concept may involve configuring the module for stackable or tray configuration at the point-of-care. By using a configurable subassembly with flexible tubing, a fluidic path may be permanently contained within a solid module body 200 during assembly with input and output sealing ports 202, 204 being held within port housings 206 which are attached to the module body 200 by hinges. Hinging of the port housings 206, relative to the module body 200, allows the input and output sealing ports 202, 204 of the fluidic pathway to be oriented for either base-tray mounting, as shown in Figure 6a, or serial connection, as shown in Figure 6b.

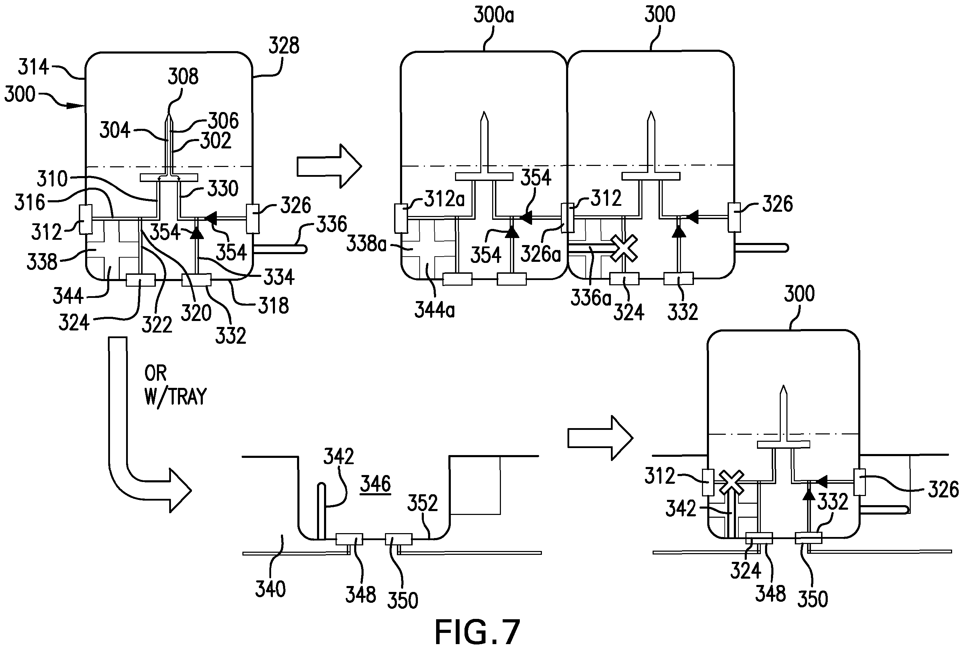

An alternate module fluidic solution allows for use of a module with alternate base tray mounting and serial connection utilizing redundant fluidics for use in either configuration and by closing off the circuit not in use at the time of use. As shown in Figure 7, each module 300 includes a vial spike 302 used to pierce a vial septum extending into the interior volume of a liquid filled drug vial to access drug therein. The vial spike 302 must be provided with sufficient length to fully pierce the septum in accessing the interior volume. The vial spike 302 includes two lumens dividing the module fluidics into separate circuits, an inlet path 304, and an outlet path 306. With the vial spike piercing the septum, the inlet path 304 and the outlet path 306 are in communication with the interior volume of the drug vial via openings formed in a distal end 308 of the vial spike 302, in similar manner to that described above in connection with the vial sike 12.

A first fluid line 310 extends from the inlet path 304 to a first sealing port 312, located on a back face 314 of the module 300, to connect the interior volume of the drug vial with the first sealing port 312. The first fluid line 310 includes a first exposed tube portion 316, which is exposed through a bottom face 318 of the module 300. A first branch line 320 extends from, and is in communication with, the first fluid line 310. The first branch line 320 includes a second exposed tube portion 322, which is exposed through the back face 314, and extends to a second sealing port 324. A third sealing port 326 is located on a front face 328 of the module 10. A second fluid line 330 connects the outlet path 306 with the third sealing port 326. A fourth sealing port 332 is located on the bottom face 318 with a second branch line 334 extending therefrom to the second fluid line 330 so as to be in communication therewith. A first male pin 336 protrudes from the front face 328 configured to pressingly engage against the second exposed tube portion 322 of a similarly-formed module connected to the module 300, as discussed below. A first port or opening 338 may be formed in the back face 314 formed to expose the second exposed tube portion 322 and to receive the first male pin 336. As shown, with two of the modules 300, 300a being serially connected, the first male pin 336a of the second module 300a is received in the first port 338 of the adjacent module 300 to press against and shut off the second exposed tube portion 322. In this manner, a continuous flow path across all drug vials of the modules 300 may be defined, outside of the second and fourth sealing ports 324, 332.

The module 300 may be utilized with a base tray 340 from which protrudes a second male pin 342 configured to pressingly engage against the first exposed tube portion 316 with the module 300 connected directly to the base tray 340. A second port or opening 344 may be formed in the bottom face 318 of the module 300 formed to expose the first exposed tube portion 316 and to receive the second male pin 342. The base tray 340 may include a nest 346 formed to insertingly receive the module 300. With the module 300 received in the nest 346, first and second outlet sealing ports 348, 350, provided on a base 352 of the nest 346, interface with the second and fourth sealing ports 324, 332, respectively, of the module 300 with the second male pin 342 being received in the second port 344 to press against and shut off the first exposed tube portion 316. In this manner, a flow path in and out of the base tray 340 may be defined, bypassing the first and third sealing ports 312, 326.

To facilitate formation of the desired fluidic circuit, one or more one-way check valves 354 may be provided to restrict flow to one-way, thereby sealing off fluid lines not in use. For example, one of the check valves 354 may be located along the second branch line 334 situated to allow only flow from the fourth sealing port 332. This restricts unwanted flow to the fourth sealing port 332 with the modules 300 being serially connected. In addition, or alternatively, one of the check valves 354 may be located along the second fluid line 330 to only allow flow from the third sealing port 326. This restricts unwanted flow to the third sealing port 326 with the module 300 mounted to the base tray 340.

As will be understood by those skilled in the art, fluidic pathway through the modules 300 may be vented as in any known manner. For example, one or both of the first and second sealing ports 312, 324 may be provided with an anti-microbial filter which allows flow of air therethrough but restricts passage of microbes therethrough. In addition, or alternatively, the fluid lines (the first fluid line 310, the first branch line 320, the second fluid line 330, the second branch line 334) may be wholly or partially formed by passages defined in the modules 300. It is preferred that the first and second exposed tube portions 316, 322 be each defined by a flexible tube to be pinchable in response to pressing engagement by the first and second male pins 336, 342, respectively.

In addition, the modules 300 may be connected in any known manner, e.g., including cooperating locking elements on opposing faces. Interengagement of the first male pin 336 with the first port 338 between adjacent modules 300 may be utilized to form a connection therebetween.

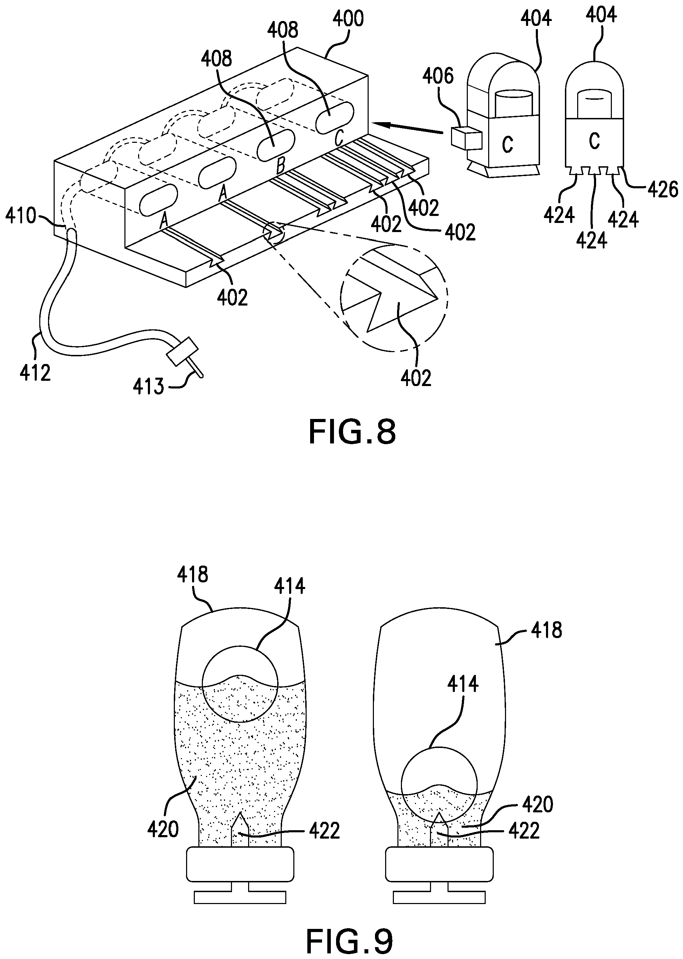

In alternate fluidic embodiments, in forming a drug delivery device, a module may interface with a base tray's fluidics through alterations to a base tray. As shown in Figure 8, wells 402 of a base tray 400 may be configured horizontally to allow for the side loading of modules 404 into the tray 400. As modules 404 are not connected in series, only one fluidic fitting would be needed to connect each of the output ports 406 of the modules 404 to corresponding inlet ports 408 of the tray 400. The inlet ports 408 of the tray 400 may be connected to a common outlet 410, e.g., by manifolded within the tray 400. The common outlet 410 may discharge into flexible tubing 412 for delivery to a needle 413 or container, such as an IV bag. Negative pressure may be applied to the common outlet 410, e.g., via the flexible tubing 412. For example, a peristaltic pump may be applied to the flexible tubing 412, with output delivered to an IV bag.

The modules 404 may be vented to atmosphere, thus, as fluid is pulled from the modules 404, air may be automatically be pulled into the respective vials to displace the fluid during transfer. As modules 404 begin to empty, their fluidic paths may be closed to prevent pulling straight from atmosphere. By way of non-limiting example, drug vials 418 of the modules 404 may be modified to each include a float valve 414, as shown in Figure 9. The float valve 414 may be a piece of pierce-able low density material held within the interior of the respective drug vial 418 floating atop the liquid drug product 420. As the drug product 420 is pulled from the bottom of the vial 418 through vial spike 422, the level of the drug product 420 lowers, eventually when the vial 418 is empty and the float 414 is all that remains within the valve the vacuum from the spike 422 will pull the float 414 on to the spike 422 sealing the spike 44, particularly the inlet and outlet lumens from the vial and any vented pathway.

Additionally, the wells 402 may be each formed with a cross-section to match the cross-section of a corresponding lock section 424 protruding from a bottom 426 of one of modules 404. This provides sliding guidance for the outlet ports 406 to interface with the inlet ports 408. In addition, the wells 402 may have non-rectangular cross-sections, e.g., a trapezoidal cross-section, with the lock sections 424 having matching cross-sections, with interengagement therebetween restricting removal of the modules 404 from the wells 402. In addition, the quantity of the wells 402 may be altered, with a corresponding quantity of the lock sections 424. This allows for controlled sequential placement of the modules 404 in a desired order. The cross-sections of the wells 402/lock sections 424 may be also, and/or alternatively, varied to also specify the desired order of the modules 404.

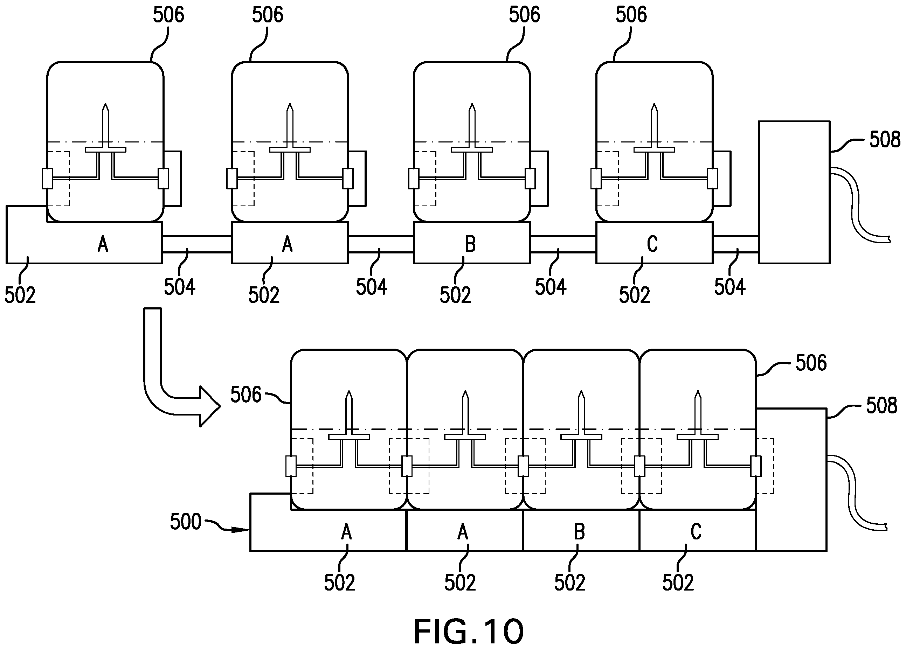

An additional embodiment that would allow the current module design to be administered through the control of a tray 500 is shown in Figure 10. The tray 500 consists of individual poke yoke platforms 502, which are joined together by telescoping rails 504. The platforms 502 have keying features on their top surfaces, which correspond to features on the underside of the modules 506. During assembly, modules 506 are attached to the correct matching platform 502 of the tray 500. When the user pushes the ends of the tray 500 together, the tray 500 is able to collapse along the telescoping rails 504, allowing the modules 506 to connect to one another creating a stack of modules 506 connected fluidically. A tubing set 508 may then be placed on the front module 506 to transfer the contents of the modules 506 to an needle or container, such as an IV bag.

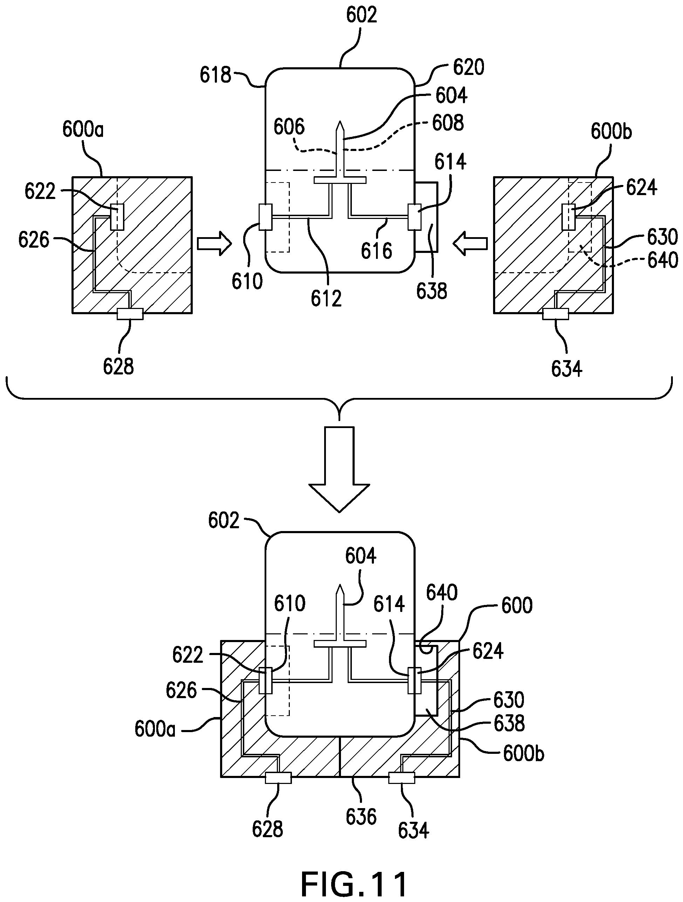

A further solution that would allow modules to interface both with a base tray and be allowed to serially connect, without altering either design may be accomplished through the use of an adapter component 600 that assembles around a module 602 altering the position of the fluid inlet and outlet ports, as shown in Figure 11. The module 602 includes a spike plate 604, formed in similar manner to the spike plate 12 described above, with inlet path 606 and outlet path 608. The module 602 further includes a first sealing port 610, which is connected to the inlet path 606 by the first fluid line 612, and second sealing port 614, which is connected to the outlet path 608 by the second fluid line 616. The first sealing port 610 is located along back face 618 of the module 602, whereas, the second sealing port 614 is located along front face 620 of the module 602. With this configuration, the first and second sealing ports 610, 614 are located on opposing faces of the module 602 in opposing directions. This configuration allows for the module 604 to be serially connected to similarly-formed modules 604 in forming a combinatorial drug delivery device.

The adapter 600 is made of a plurality of pieces 600a, 600b, including possibly two halves, representing an input side and an output side. The adapter 600 may be mounted to the module 602 by joining the pieces 600a, 600b. For example, the halves 600a, 600b of the adapter 600 may be placed around the module 602 and pushed together to cause them to permanently join together with plastic snap features around the module 602.

The adapter 600 includes first and second inner ports 622, 624. With the adapter 600 mounted to the module 602, the first inner port 622 is located to align with the first sealing port 610, and the second inner port 624 is located to align with the second sealing port 614. A first secondary fluid line 626 connects the first inner port 622 with a first outlet port 628. A second secondary fluid line 630 connects the second inner port 632 with a second outlet port 634. The first and second outlet ports 628, 634 are located on a common face 636 of the adapter 600 to face in a common direction, transverse to the facing directions of the first and second sealing ports 610, 614. This arrangement allows the module 602 to be fluidically connected to a base tray, in the adapter 600. Thus, the module 602, without the adapter 600, may be serially connected, and, with the adapter 600, may be used with a base tray.

Components of the adapter 600 may be split amongst the pieces 600a, 600b, including being evenly split. For example, the first inner port 622, the first secondary fluid line 626, and the first outlet port 628 may be located on one of the pieces 600a, with the second inner port 624, the second secondary fluid line 630, and the second outlet port 634 being located on a second piece 600b. This allows for fluid pathways be wholly contained in each of the pieces 600a, 600b without interruption thereof. In addition, one or more mating parts may be formed on the adapter 600 and the module 602 to enhance the connection therebetween. For example, one or more protrusions 638 may be formed on the adapter 600 and/or the module 602 formed to be nestingly received in corresponding hollows 640 formed in the adapter 600 and/or the module 602.

## Claims

1. A module (10) for use in a combinatorial drug device (54), the module (10) formed to accommodate a drug vial (18) sealed by a septum (14), the module (10) comprising:
a vial spike (12) formed to pierce the septum (14) of the drug vial (18) accommodated by the module (10), wherein the vial spike (12) includes an inlet path (22) and a separate outlet path (24);
a first sealing port (28);
a first fluid line (26) connecting the inlet path (22) with the first sealing port (28);
a fourth sealing port (44); and,
a third fluid line (46) connecting the outlet path (24) with the fourth sealing port (44);
**characterized by**,
a first branch line (32) extending from, and in communication with, the first fluid line (26), the first branch line (32) including an exposed tube portion (34);
a second sealing port (36);
a third sealing port (38);
a second fluid line (42) connecting the second sealing port (36) with the third sealing port (38), wherein the first branch line (32) extends to, and is in communication with, the second fluid line (42); and,
a male pin (48) formed to pressingly engage against the exposed tube portion (34) of a similarly-formed module (10) connected directly to the module (10).

2. A module (10) as in claim 1, further comprising at least one collapsible gas chamber (52) in communication with or more of: the first fluid line (26), the second fluid line (42), and the third fluid line (46).

3. A combinatorial drug delivery device (54) comprising:
a plurality of modules (10) formed in accordance with claim 1 serially connected to form a combination of the modules (10) with a first of the modules (10) defining a terminus thereof; and,
a pump module (56) connected to the first module (10), the pump module (56) including a first inlet sealing port (58) interfacing with the fourth sealing port (44) of the first module (10), a second inlet sealing port (60) interfacing with the third sealing port (38) of the first module (10), a first outlet port (62), and a pump (64), wherein, an outlet passageway (66) is defined between the first inlet sealing port (58) and the first outlet port (62), with the pump (64) being configured to draw liquid drug into the outlet passageway (66) and to urge the drawn liquid drug from the first outlet port (62).

4. A combinatorial drug delivery device (54) as in claim 3, wherein the pump module (56) includes a vented passageway (68) in communication with the second inlet sealing port (60).

5. A combinatorial drug delivery device (54) as in claim 3, wherein the pump module (56) includes at least one collapsible gas chamber (52) in communication with the second inlet sealing port (60).

6. A module (300) for use in a combinatorial drug device, the module (300) formed to accommodate a drug vial sealed by a septum, the module (300) comprising:
a vial spike (302) formed to pierce the septum of the drug vial accommodated by the module (300), wherein the vial spike includes an inlet path (304) and a separate outlet path (306);
a first sealing port (312);
a first fluid line (310) connecting the inlet path (304) with the first sealing port (312)[[,]]
a third sealing port (326); and,
a second fluid line (330) connecting the outlet path (306) with the third sealing port (326);
**characterized by**,
the first fluid line (310) including a first exposed tube portion (316);
a second sealing port (324);
a first branch line (320) extending from, and in communication with, the first fluid line (310), the first branch line (320) extending to the second sealing port (324), the first branch line (320) including a second exposed tube portion (322);
a fourth sealing port (332);
a second branch line (334) extending from, and in communication with, the second fluid line (330), the second branch line (334) extending to the fourth sealing port (332); and.
a male pin (336) formed to pressingly engage against the second exposed tube portion (322) of a similarly-formed module (300) connected directly to the module (300).

7. A combination comprising:
a module (300) as in claim 6; and,
a base tray (340) including a second male pin (342) formed to pressingly engage against the first exposed tube portion (316) with the module (300) connected directly to the base tray (340).

## Patentansprüche

1. Modul (10) zur Verwendung in einer kombinatorischen Arzneimittelvorrichtung (54), wobei das Modul (10) gebildet ist, um ein Arzneimittelfläschchen (18), das mit einem Septum (14) abgedichtet ist, aufzunehmen, wobei das Modul (10) Folgendes umfasst:
einen Fläschchendorn (12), der gebildet ist, um das Septum (14) des Arzneimittelfläschchens (18), das von dem Modul (10) aufgenommen ist, zu durchstechen, wobei der Fläschchendorn (12) einen Einlassweg (22) und einen separaten Auslassweg (24) einschließt;
einen ersten abdichtenden Anschluss (28);
eine erste Fluidleitung (26), die den Einlassweg (22) mit dem ersten abdichtenden Anschluss (28) verbindet;
einen vierten abdichtenden Anschluss (44); und
eine dritte Fluidleitung (46), die den Auslassweg (24) mit dem vierten abdichtenden Anschluss (44) verbindet;
**gekennzeichnet durch**
eine erste Zweigleitung (32), die sich von und in Verbindung mit der ersten Fluidleitung (26) erstreckt, wobei die erste Zweigleitung (32) einen freiliegenden Rohrabschnitt (34) einschließt;
einen zweiten abdichtenden Anschluss (36);
einen dritten abdichtenden Anschluss (38);
eine zweite Fluidleitung (42), die den zweiten abdichtenden Anschluss (36) mit dem dritten abdichtenden Anschluss (38) verbindet, wobei sich die erste Zweigleitung (32) zu der zweiten Fluidleitung (42) erstreckt und mit dieser in Verbindung steht; und
einen Steckstift (48), der gebildet ist, um pressend gegen den freiliegenden Rohrabschnitt (34) eines ähnlich gebildeten Moduls (10), das direkt mit dem Modul (10) verbunden ist, in Eingriff zu stehen.

2. Modul (10) nach Anspruch 1, ferner mindestens eine zusammenklappbare Gaskammer (52) in Verbindung mit oder mehreren der Folgenden umfassend: der ersten Fluidleitung (26), der zweiten Fluidleitung (42) und der dritten Fluidleitung (46).

3. Kombinatorische Arzneimittel-Abgabevorrichtung (54), umfassend:
eine Vielzahl von Modulen (10), die gemäß Anspruch 1 gebildet sind, die in Reihe verbunden sind, um eine Kombination der Module (10) zu bilden, wobei ein erstes der Module (10) einen Endpunkt davon definiert; und
ein Pumpenmodul (56), das mit dem ersten Modul (10) verbunden ist, wobei das Pumpenmodul (56) einen ersten abdichtenden Einlassanschluss (58), der mit dem vierten abdichtenden Anschluss (44) des ersten Moduls (10) gekoppelt ist, einen zweiten abdichtenden Einlassanschluss (60), der mit dem dritten abdichtenden Anschluss (38) des ersten Moduls (10) gekoppelt ist, einen ersten Auslassanschluss (62) und eine Pumpe (64) einschließt, wobei ein Auslasskanal (66) zwischen dem ersten abdichtenden Einlassanschluss (58) und dem ersten Auslassanschluss (62) definiert ist, wobei die Pumpe (64) konfiguriert ist, um flüssiges Arzneimittel in den Auslasskanal (66) zu saugen und das eingesaugte flüssige Arzneimittel aus dem ersten Auslassanschluss (62) herauszupressen.

4. Kombinatorische Arzneimittel-Abgabevorrichtung (54) nach Anspruch 3, wobei das Pumpenmodul (56) einen belüfteten Kanal (68) in Verbindung mit dem zweiten abdichtenden Einlassanschluss (60) einschließt.

5. Kombinatorische Arzneimittel-Abgabevorrichtung (54) nach Anspruch 3, wobei das Pumpenmodul (56) mindestens eine zusammenklappbare Gaskammer (52) in Verbindung mit dem zweiten abdichtenden Einlassanschluss (60) einschließt.

6. Modul (300) zur Verwendung in einer kombinatorischen Arzneimittel-Abgabevorrichtung, wobei das Modul (300) gebildet ist, um ein Arzneimittelfläschchen, das mit einem Septum abgedichtet ist, aufzunehmen, wobei das Modul (300) Folgendes umfasst:
einen Fläschchendorn (302), der gebildet ist, um das Septum des Arzneimittelfläschchens, das von dem Modul (300) aufgenommen ist, zu durchstechen, wobei der Fläschchendorn einen Einlassweg (304) und einen separaten Auslassweg (306) einschließt;
einen ersten abdichtenden Anschluss (312);
eine erste Fluidleitung (310), die den Einlassweg (304) mit dem ersten abdichtenden Anschluss (312) verbindet;
einen dritten abdichtenden Anschluss (326); und
eine zweite Fluidleitung (330), die den Auslassweg (306) mit dem dritten abdichtenden Anschluss (326) verbindet;
**gekennzeichnet durch**
die erste Fluidleitung (310), die einen ersten freiliegenden Rohrabschnitt (316) einschließt;
einen zweiten abdichtenden Anschluss (324);
eine erste Zweigleitung (320), die sich von und in Verbindung mit der ersten Fluidleitung (310) erstreckt, wobei sich die erste Zweigleitung (320) zu dem zweiten abdichtenden Anschluss (324) erstreckt, wobei die erste Zweigleitung (320) einen zweiten freiliegenden Rohrabschnitt (322) einschließt;
einen vierten abdichtenden Anschluss (332);
eine zweite Zweigleitung (334), die sich von und in Verbindung mit der zweiten Fluidleitung (330) erstreckt, wobei sich die zweite Zweigleitung (334) zu dem vierten abdichtenden Anschluss (332) erstreckt; und
einen Steckstift (336), der gebildet ist, um pressend gegen den zweiten freiliegenden Rohrabschnitt (322) eines ähnlich gebildeten Moduls (300), das direkt mit dem Modul (300) verbunden ist, in Eingriff zu stehen.

7. Kombination, umfassend:
ein Modul (300) nach Anspruch 6; und
eine Basiswanne (340), die einen zweiten Steckstift (342) einschließt, der gebildet ist, um pressend gegen den ersten freiliegenden Rohrabschnitt (316) mit dem Modul (300), das direkt mit der Basiswanne (340) verbunden ist, in Eingriff zu kommen.

## Revendications

1. Module (10) destiné à être utilisé dans un dispositif combinatoire de médicaments (54), le module (10) étant formé pour accueillir un flacon de médicament (18) scellé par un septum (14), le module (10) comprenant:
une pointe de flacon (12) formée pour percer le septum (14) du flacon de médicament (18) accueilli par le module (10), dans lequel la pointe de flacon (12) inclut un chemin d'entrée (22) et un chemin de sortie séparé (24);
un premier orifice de scellement (28);
une première conduite de fluide (26) connectant le chemin d'entrée (22) au premier orifice de scellement (28);
un quatrième orifice de scellement (44); et,
une troisième conduite de fluide (46) connectant le chemin de sortie (24) au quatrième orifice de scellement (44);
**caractérisé par**,
une première conduite de ramification (32) s'étendant depuis, et en communication avec, la première conduite de fluide (26), la première conduite de ramification (32) incluant une partie de tube exposée (34);
un deuxième orifice de scellement (36);
un troisième orifice de scellement (38);
une deuxième conduite de fluide (42) connectant le deuxième orifice de scellement (36) au troisième orifice de scellement (38), dans lequel la première conduite de ramification (32) s'étend vers, et en communication avec, la deuxième conduite de fluide (42); et
une broche mâle (48) formée pour se mettre en prise par pression contre la partie de tube exposée (34) d'un module formé de manière similaire (10) connecté directement au module (10).

2. Module (10) selon la revendication 1, comprenant en outre au moins une chambre à gaz escamotable (52) en communication avec ou plusieurs parmi: la première conduite de fluide (26), la deuxième conduite de fluide (42) et la troisième conduite de fluide (46).

3. Dispositif d'administration combinatoire de médicaments (54) comprenant:
une pluralité de modules (10) formés conformément à la revendication 1 connectés en série pour former une combinaison des modules (10), un premier des modules (10) définissant une terminaison de ceux-ci; et
un module de pompe (56) connecté au premier module (10), le module de pompe (56) incluant un premier orifice de scellement d'entrée (58) couplé avec le quatrième orifice de scellement (44) du premier module (10), un deuxième orifice de scellement d'entrée (60) couplé avec le troisième orifice de scellement (38) du premier module (10), un premier orifice de sortie (62) et une pompe (64), dans lequel un passage de sortie (66) est défini entre le premier orifice de scellement d'entrée (58) et le premier orifice de sortie (62), la pompe (64) étant configurée pour aspirer un médicament liquide dans le passage de sortie (66) et pour pousser le médicament liquide aspiré depuis le premier orifice de sortie (62).

4. Dispositif d'administration combinatoire de médicaments (54) selon la revendication 3, dans lequel le module de pompe (56) inclut un passage aéré (68) en communication avec le deuxième orifice de scellement d'entrée (60).

5. Dispositif d'administration combinatoire de médicaments (54) selon la revendication 3, dans lequel le module de pompe (56) inclut au moins une chambre à gaz escamotable (52) en communication avec le deuxième orifice de scellement d'entrée (60).

6. Module (300) destiné à être utilisé dans un dispositif combinatoire de médicaments, le module (300) étant formé pour accueillir un flacon de médicament scellé par un septum, le module (300) comprenant:
une pointe de flacon (302) formée pour percer le septum du flacon de médicament accueilli par le module (300), dans lequel la pointe de flacon inclut un chemin d'entrée (304) et un chemin de sortie séparé (306);
un premier orifice de scellement (312);
une première conduite de fluide (310) connectant le chemin d'entrée (304) au premier orifice de scellement (312),
un troisième orifice de scellement (326); et,
une deuxième conduite de fluide (330) connectant le chemin de sortie (306) au troisième orifice de scellement (326);
**caractérisé par**,
une première conduite de fluide (310) qui inclut une première partie de tube exposée (316);
un deuxième orifice de scellement (324);
une première conduite de ramification (320) s'étendant depuis, et en communication avec, la première conduite de fluide (310), la première conduite de ramification (320) s'étendant vers le deuxième orifice de scellement (324), la première conduite de ramification (320) incluant une seconde partie de tube exposée (322);
un quatrième orifice d'étanchéité (332);
une seconde conduite de ramification (334) s'étendant depuis, et en communication avec, la deuxième conduite de fluide (330), la deuxième conduite de ramification (334) s'étendant vers le quatrième orifice de scellement (332); et
une broche mâle (336) formée pour se mettre en prise par pression contre la seconde partie de tube exposée (322) d'un module formé de manière similaire (300) connecté directement au module (300).

7. Combinaison comprenant:
un module (300) selon revendication 6; et,
un plateau de base (340) incluant une seconde broche mâle (342) formée pour se mettre en prise par pression, contre la première partie de tube exposée (316), avec le module (300) connecté directement au plateau de base (340).
